**Europäisches Patentamt**

**European Patent Office**

**Office européen des brevets**

(19)

(11) Veröffentlichungsnummer: **0 131 842**
**B1**

(12) **EUROPÄISCHE PATENTSCHRIFT**

(45) Veröffentlichungstag der Patentschrift:
**10.12.86**

(51) Int. Cl.⁴: **C 08 G 59/50, C 07 D 233/54**

(21) Anmeldenummer: **84107711.8**

(22) Anmeldetag: **03.07.84**

(54) **Flüssiger Epoxidharzhärter und Verfahren zu dessen Herstellung.**

(30) Priorität: **06.07.83 DE 3324339**

(43) Veröffentlichungstag der Anmeldung:
**23.01.85 Patentblatt 85/4**

(45) Bekanntmachung des Hinweises auf die Patenterteilung:
**10.12.86 Patentblatt 86/50**

(84) Benannte Vertragsstaaten:
**BE DE FR GB NL**

(56) Entgegenhaltungen:
**FR - A - 2 074 845**
**GB - A - 2 067 432**

**CHEMICAL ABSTRACTS, Band 97, Nr. 22, 29. November 1982, Seite 45, Nr. 183442r, Columbus, Ohio, US;**
**CHEMICAL ABSTRACTS, Band 97, Nr. 22, 29. November 1982, Seite 45, Nr. 183444t, Columbus, Ohio, US;**

(73) Patentinhaber: **BASF Aktiengesellschaft,**
**Carl-Bosch-Strasse 38, D-6700 Ludwigshafen (DE)**

(72) Erfinder: **Dockner, Toni, Dr., Grossgasse 6,**
**D-6701 Meckenheim (DE)**
Erfinder: **Frank, Anton, Bannwasserstrasse 72,**
**D-6700 Ludwigshafen (DE)**

## Beschreibung

Die Erfindung betrifft flüssige Epoxidharzhärter auf Basis alkylierter Imidazole.

Die Dehydrierung von △2-Imidazolinen an verschiedenen Katalysatoren zu den entsprechenden substituierten Imidazolen ist bekannt und wird beispielsweise in EP 0 000 208, DE-A-1 952 991, DE-C-2 106 877, DE-A-2 733 466, US-A-2 847 417, US-A-3 037 082, US-A-3 177 223 und SU-A-201 418 beschrieben.

Die Verwendung von Imidazolverbindungen, wie 2-Ethyl-4-methylimidazol, 2-Cyclohexyl-4-methylimidazol und 2-Octyl-4-hexylimidazol als Epoxidharzhärter war aus der DE-B-1 301 135 bekannt. In diesem Dokument wird zwar als besonders bevorzugtes flüssiges Imidazol das 2-Ethyl-4-methylimidazol genannt, tatsächlich schmilzt jedoch reines 2-Ethyl-4-methylimidazol bei 45°C (vgl. Hofmann, Imidazoles and Derivatives, Seite 328) und ist bei Zimmertemperatur eine erstarrte Kristallschmelze.

Will man nach DE-B-1 301 135 2-Ethyl-4-methylimidazol flüssig einsetzen, so muss man die Kristalle aufschmelzen und die Schmelze bei Temperaturen von über 40°C handhaben.

Mischt man im gleichen Gewichtsverhältnis reines 2-Ethyl-4-methylimidazol mit Imidazol oder anderen Alkylimidazolen wie 2-Methyl-, 2-Isopropyl-, 4-Methylimidazol usw., selbst mit dem flüssigen 1-Methylimidazol oder mit dem Ausgangsstoff 2-Ethyl-4--methylimidazolin, der ebenfalls flüssig ist, so kristallisieren diese Mischungen nach einigen Tagen entweder zu einem festen Kristallklotz oder zu Kristallmaischen.

Aufgabe der vorliegenden Erfindung ist es, einen Epoxidharzhärter auf Basis alkylierter Imidazole aufzuzeigen, der bei Raumtemperatur flüssig und möglichst niedrigviskos ist, auch bei Temperaturen bis zu 0°C flüssig bleibt und gute Härtereigenschaften aufweist.

Es wurde nun gefunden, dass dieses Ziel überraschenderweise dadurch erreicht wird, dass man als Epoxidharzhärter ein Gemisch aus 2-Ethyl-4-methylimidazol, Ethyldimethylimidazol sowie Diethylmethylimidazolisomeren verwendet.

Gegenstand der vorliegenden Erfindung ist ein flüssiger Epoxidharzhärter, der dadurch gekennzeichnet ist, dass er besteht aus einem Gemisch folgender alkylierter Imidazole

75 bis 90 Gew.-% 2-Ethyl-4-methylimidazol,
1 bis 6 Gew.-% 2-Ethylimidazol,
2 bis 4 Gew.-% 4-Methylimidazol,
0,5 bis 1,5 Gew.-% 2,4-Dimethylimidazol,
0,5 bis 1,5 Gew.-% 2-Methyl-4-ethylimidazol,
1 bis 10 Gew.-% 2-Ethyl-4,5-dimethylimidazol und
1 bis 10 Gew.-% 2,5-Diethyl-4-methylimidazol, bzw. 2,4-Diethyl-5-methyl imidazol.

Der erfindungsgemässe Epoxidharzhärter bleibt bei Raumtemperatur, sowie bei Temperaturen bis zum Gefrierpunkt des Wassers flüssig, weist damit vorteilhaftere Verarbeitungseigenschaften und Härtereigenschaften auf, die denen des 2-Ethyl-4-methylimidazols zumindest gleichwertig sind.

Die Herstellung der erfindungsgemässen flüssigen Epoxidharzhärter kann durch Vermischen der oben genannten, einzelnen reinen Verbindungen oder deren Gemischen erfolgen.

Besonders vorteilhaft können die erfindungsgemässen Gemische durch Destillation der bei der Dehydrierung von 2-Ethyl-4-methylimidazolin bei Temperaturen über 400°C, vorzugsweise 400 bis 550°C, an zinkoxidhaltigen Katalysatoren gebildeten Produkte gewonnen werden.

Das erfindungsgemässe Gemisch ist bei Raumtemperatur eine zähflüssige Masse, die auch nach Animpfen mit Kristallen und längerer Aufbewahrung im Kühlschrank bei 0 bis 5°C nicht kristallisiert.

Die Dehydrierung von 2-Ethyl-4-methylimidazolin kann wie in EP 0 000 208 beschrieben durchgeführt werden. Erhöht man die Dehydriertemperatur auf 400 bis 500°C, so kann der Anteil der 2-Alkyl-, 4-Alkyl-, 2,4,5-Trialkylimidazole erhöht werden. Das Reaktionsgemisch kann, wie es aus der Dehydrierung anfällt, über eine Destillationsapparatur mit nur einem theoretischen Boden (Claisenaufsatz) destilliert werden. Bevorzugt wird bei Normaldruck destilliert und die Fraktion von 250 bis 274°C (1013 mbar) gewonnen. Man kann aber auch unter vermindertem Druck destillieren. Eine typische Zusammensetzung eines bei Raumtemperatur flüssigen Härtersystems ist z.B.

85 % 2-Ethyl-4-methylimidazol
2,5 % 2-Ethylimidazol
1,5 % 4-Methylimidazol
0,5 % 2,4-Dimethylimidazol
0,5 % 2-Methyl-4-ethylimidazol
6 % 2-Ethyl-4,5-dimethylimidazol und
4 % 2,5(4)-Diethyl-4(5)-methylimidazol.

Die erfindungsgemässen flüssigen Epoxidharzhärter lassen sich sehr vorteilhaft verarbeiten und zusammen mit den üblichen Epoxidharzen verwenden. Das Mengenverhältnis zwischen Epoxidharz und erfindungsgemässem Epoxidharzhärter kann bei der Anwendung innerhalb eines weiten Bereichs schwanken. Der Epoxidharzhärter kann beispielsweise in einer Menge von 0,1 bis 50, vorzugsweise in einer Menge von 5 bis 30 Gew.-% der Epoxidverbindung verwendet werden.

Als Epoxidharz kommen die üblichen Epoxidverbindungen in Frage, die im Durchschnitt mehr als eine Epoxidgruppe je Molekül enthalten.

Die Epoxidverbindungen können bekanntermassen gesättigt oder ungesättigt, aliphatisch, cycloaliphatisch, aromatisch oder heterocyclisch sein sowie gegebenenfalls Substituenten aufweisen, die nicht an der Reaktion teilnehmen, wie Halogenatome, Hydroxylgruppen und Etherreste.

Bevorzugte Epoxidverbindungen sind Glycidylether von mehrwertigen Phenolen, z.B. Diphenylolalkane, beispielsweise Diphenylolpropan, Diphenylolethan und Diphenylolmethan, Diphenylolsulfon, Hydrochinon, Resorcin, Dihydroxydiphenyl, Dihydroxynaphthalin, oder mehrwertige Phenole, wie Novolacke und Resole, die durch Kondensation von Phenol und Formaldehyd hergestellt werden. Am besten geeignet sind Glycidylpolyether von 2,2-Bis-(4-hydroxyphenyl)-propan mit einem Molekulargewicht zwischen 340 und 4000.

Weitere brauchbare Epoxidverbindungen sind Polyepoxyalkylether von aliphatischen Polyhydroxyverbindungen, wie etwa Ethylenglykol, Glycerin, Trimethylolpropan und Pentaerythrit, Polyepoxyalkylester von mehrbasischen Carbonsäuren, z.B. Diglycidylester von Phthalsäure, Terephthalsäure und Adipinsäure, und Polyglycidylester von polymeren ungesättigten Fettsäuren, beispielsweise der Diglycidylester der dimerisierten Linolsäure; epoxidiertes Leinöl oder Sojabohnenöl; epoxydierte Diene, wie Diepoxybutan und expoxydiertes Vinylcyclohexan; Diepoxyalkylether, in denen zwei Epoxyalkylgruppen an nur ein Sauerstoffatom gebunden sind, wie Diglycidylether; und Polyepoxyverbindungen, die durch Epoxydierung von Cyclohexenderivaten erhalten werden, wie z.B. der (3,4-Epoxy-6-methylcyclohexyl)-methylester der 3,4-Epoxy-6-methyl-cyclohexancarbonsäure.

Die erfindungsgemässen Epoxidharzhärter lassen sich in an sich bekannter Weise auch gemeinsam mit anderen für die Epoxydpolyadduktbildung bekannter Umsetzungskomponente verwenden, z.B. mit Phenolen, Mercaptanen, Triphenylphosphin, Triphenylarsin, Triphenylstibin, Aminen, Aminsalzen und quaternären Ammoniumsalzen.

Beispiele für geeignete und für den genannten Zweck bekannte Amine, die gemeinsam mit den erfindungsgemässen Epoxidharzhärtern verwendet werden können, sind Benzyldimethylamin, Dicyandiamid, p,p'-Bis-(dimethylaminophenyl)-methan, Dimethylethanolamin, Morpholin, Dimethylaminopropylamin, Metaphenylendiamin, Polyalkylenpolyamine, wie Diethylentriamin und Gemische der oben erwähnten Amine. Die Salze dieser Amine können aus einer anorganischen oder organischen Säure und einem Amin gebildet sein, wie beispielsweise die Hydrochloride, die Sulfate und die Acetate der oben beschriebenen tertiären Amine. Beispiele für quaternäre Verbindungen sind: Benzyltrimethylammoniumchlorid, Phenyltributylammoniumchlorid, Cyclohexyltributylammoniumsulfat, Benzyltrimethylammoniumsulfat, Benzyltrimethylammoniumborat, Diphenyldioctylammoniumchlorid und Gemische derselben.

Diese zusätzlichen Amine können in an sich bekannter Weise in Mengen von 0,1 bis 25 Gew.-%, vorzugsweise von 1 bis 5 Gew.-%, bezogen auf die Epoxidverbindungen, verwendet werden.

Das Ausreagieren wird nach Vermischen der Epoxidverbindung mit dem Epoxidharzhärter durch Erhitzen des so erhaltenen Gemisches bewirkt. Dem Gemisch können vor der Umsetzung verschiedene Zusatzstoffe zugefügt werden, wie Lösungsmittel, Verdünnungsmittel, Pigmente, Füllstoffe, faserartige Produkte, Farbstoffe, Harze, Weichmacher und nichtflüchtige Streckmittel. Ist die Epoxidverbindung eine relativ viskose Flüssigkeit oder eine feste Substanz, dann kann man die Komponenten schon beim Vermischen erwärmen oder ein Lösungsmittel verwenden, z.B. Benzol, Toluol, Cyclohexan, Ketone, Ether, Ester, Nitrile.

Weiterhin können in an sich bekannter Weise Monoepoxyverdünnungsmittel verwendet werden, wie Butylglycidylether, Phenylglycidylether und Monoglycidylester. Monoepoxyverdünnungsmittel nehmen an der Umsetzung teil und können im allgemeinen in Mengen von bis zu 20 Gew.-% der Epoxydverbindung verwendet werden. Es können auch inerte, nichtflüchtige Streckmittel verwendet werden, wie Kohlenteere, Kohlenteerpeche, Asphalte, Kiefernpech, Terpentinöl, Schmierölfraktionen und deren aromatische Extrakte, sowie Schmierölraffinate.

Die während der Umsetzung anzuwendende Temperatur kann innerhalb eines weiten Bereiches schwanken und liegt im allgemeinen zwischen ungefähr 40 und 300°C, vorzugsweise zwischen 50 und 250°C.

Die mit den erfindungsgemässen Epoxidharzhärtern erhaltenen Formkörper besitzen eine überraschend hohe Formbeständigkeit in der Wärme und auch bei erhöhter Temperatur noch sehr gute Festigkeitseigenschaften. Ausserdem weisen sie eine grosse Beständigkeit gegenüber kochendem Wasser und starken Lösungsmitteln und anderen Chemikalien auf, so dass sie besonders wertvoll für die Herstellung von Verklebungen, Schichtstoffen und anderen Formkörpern, wie Einkapselungen von elektrischen Ausrüstungen und gegebenenfalls als Überzüge sind. Die erfindungsgemässen Epoxidharzhärter eignen sich auch als Cokatalysatoren in Verbindung mit Aminen, insbesondere zum Überziehen von hitzebeständigen Materialien, wie Glas- oder Kohlenstoff-Fasern mit Epoxidharzen.

Ein weiterer Vorteil der erfindungsgemässen Härter beruht auf der Tatsache, dass die damit bereiteten Massen bei mässig hoher Temperatur zur Reaktion gebracht werden können, so dass bei ihrer Verwendung keine nachteiligen Wirkungen auf das hitzeempfindliche Material, auf welches sie aufgebracht werden, zu befürchten ist.

Die in den Beispielen genannten Teile und Prozente sind, sofern nicht anders angegeben, Gewichtsteile und Gewichtsprozente.

*Beispiel 1*

In einem Wirbelreaktor, der 200 Teile eines aus ZnO/Al$_2$O$_3$ bestehenden Dehydrierungskatalysators (wie in der EP 0 000 208 näher beschrieben) enthält, werden stündlich 100 Teile 2-Ethyl-4-methylimidazolin, die in einem elektrisch beheizten Quarzverdampfer vorverdampft und mit 5000 Teilen Stickstoff verdünnt wurden, bei 400°C dehydriert. Der Reaktoraustrag wird in einer Destillatonsapparatur ohne Verwendung einer Destillationskolonne bei Normaldruck oder unter Vakuum destilliert. Man erhält pro 100 Teile eingesetztem 2-Ethyl-4-methylimidazolin 92 Teile eines Imidazolgemisches, das nach GC-Analyse

88,6 % 2-Ethyl-methylimidazol
  1,4 % 2-Ethylimidazol
  2,0 % 4-Methylimidazol
  2,0 % 2-Ethyl-4,5-dimethylimidazol
  5,0 % 2,5(4)-Diethyl-4(5)-methylimidazol
  0,5 % 2,4-Dimethylimidazol und
  0,5 % 2-Methyl-4-ethylimidazol

enthält und bei 250 bis 274°C siedet.

Als Vorlauf werden 5 Teile nicht umgesetztes 2-Ethyl-4-methylimidazolin abgetrennt und in das Verfahren zurückgeführt. Als Destillationsrückstand verbleiben 3 Teile einer schwarzen Masse. Das Destillat ist eine viskose Flüssigkeit, die auch nach mehr als 30 Tagen im Kühlschrank und täglichem Reiben mit dem Glasstab nicht kristallisiert.

*Beispiel 2*

Arbeitet man, wie in Beispiel 1 beschrieben, bei einer Temperatur von 500°C im Wirbelreaktor, so erhält man pro 100 Teile eingesetztem 2-Ethyl-4--methylimidazolin 89 Teile eines Imidazolgemisches, das nach GC-Analyse

78,5 % 2-Ethyl-4-methylimidazol
 3,5 % 2-Ethylimidazol
 3,5 % 4-Methylimidazol
   9 % 2-Ethyl-4,5-dimethylimidazol
 3,5 % 2,5(4)-Diethyl-4(5)-methylimidazol
   1  % 2,4-Dimethylimidazol und
   1  % 2-Methyl-4-ethylimidazol

enthält und bei 20 bis 25 mbar bei 160 bis 180°C siedet.

Als Vorlauf werden 3 Teile nichtumgesetztes 2-Ethyl-4-methylimidazolin zurückgewonnen. Als Destillationsrückstand verbleiben 8 Teile einer schwarzen Masse. Das Destillat bleibt auch beim Stehen im Kühlschrank über 30 Tage flüssig.

*Vergleichsbeispiel*
8,5 Teile 2-Ethyl-4-methylimidazol, 99,9%ig
0,5 Teile 2-Ethyl-4-methylimidazolin
0,5 Teile 1-Methylimidazol
0,5 Teile 4-Methylimidazol

werden zusammengeschmolzen und die Schmelze im Kühlschrank aufbewahrt. Nach zweitägigem Stehen beginnt das Gemisch zu kristallisieren, nach weiteren zwei Tagen ist die Mischung durchkristallisiert.


**Patentanspruch**

Flüssiger Epoxidharzhärter, dadurch gekennzeichnet, dass er besteht aus einem Gemisch folgender alkylierter Imidazole

75 bis 90  Gew.-% 2-Ethyl-4-methylimidazol,
 1  bis 6   Gew.-% 2-Ethylimidazol,
 2  bis 4   Gew.-% 4-Methylimidazol,
0,5 bis 1,5 Gew.-% 2,4-Dimethylimidazol,
0,5 bis 1,5 Gew.-% 2-Methyl-4-ethylimidazol,
 1  bis 10  Gew.-% 2-Ethyl-4,5-dimethylimidazol
            und
 1  bis 10  Gew.-% 2,5-Diethyl-4-methylimidazol,
            bzw. 2,4-Diethyl-5-methyl
            imidazol.


**Claim**

A liquid epoxy resin hardener which consists of a mixture of the following alkylated imidazoles:
from 75  to 90  % by weight of 2-ethyl-4-methylimidazole,
from  1  to  6  % by weight of 2-ethylimidazole,
from  2  to  4  % by weight of 4-methylimidazole,
from 0.5 to  1.5% by weight of 2,4-dimethylimidazole,
from 0.5 to  1.5% by weight of 2-methyl-4--ethylimidazole,
from  1  to 10  % by weight of 2-ethyl-4,5-dimethylimidazole, and
from  1  to 10  % by weight of 2,5-diethyl-4--methylimidazole, or 2,4--diethyl-5-methylimidazole.


**Revendication**

Agent durcissant liquide pour résines époxydes, caractérisé par le fait qu'il est constitué d'un mélange des imidazoles alkylés suivants:
75  à 90  % en poids 2-éthyl-4-méthylimidazole,
 1  à  6  % en poids 2-éthylimidazole,
 2  à  4  % en poids 4-méthylimidazole,
0,5 à  1,5% en poids 2,4-diméthylimidazole,
0,5 à  1,5% en poids 2-méthyl-4-éthylimidazole,
 1  à 10  % en poids 2-éthyl-4,5-diméthyl-imidazole et
 1  à 10  % en poids 2,5-diéthyl-4-méthyl-imidazole, ou 2,4-diéthyl-5-méthyl-imidazole.